# EUROPEAN PATENT SPECIFICATION

(11) **EP 0 775 500 B1**
(45) Date of publication and mention of the grant of the patent: **28.01.2004**
(21) Application number: 96203274.4
(22) Date of filing: 22.11.1996
(51) Int. Cl.: A61M 25/01

(54) **Medical device visible when using magnetic resonance imaging**
Medizinische Vorrichtung sichtbar während des Gebrauchs einer magnetischen Resonanz-Bilderzeugung
Dispositif médical visible quant on utilise l'imagerie par résonance magnétique

(30) Priority: 23.11.1995 NL 1001736
(43) Date of publication of application: 28.05.1997
(73) Proprietor: Cordis Corporation, Miami Lakes Florida 33014 (US)
(72) Inventor: Weber, Jan, 9302 ER Roden (NL); Bakker, Christianus Johannes Gerardus, 3583 TJ Utrecht (NL)
(74) Representative: 't Jong, Bastiaan Jacob

(56) References cited:
- EP-A- 0 702 976
- WO-A-87/04080
- WO-A-94/23782
- WO-A-95/21566

## Description

The invention relates to a medical device such as a catheter, balloon, graft and stent, which is introduced into the body of a patient either temporarily or for a longer period of time for the purpose of treatment and/or investigation. More particular, this invention relates to a device which can be made visible under NMR-conditions.

From WO87/04080 it is known to make such a device visible by means of ferro or paramagnetic materials. The ferro or paramagnetic materials disturb the magnetic field in the NMR device in a controlled manner, as a result of which they become visible on the accessory imaging device.

The object of the invention is to further improve such a medical device, in particular to improve its visibility when using magnetic resonance imaging.

This aim is achieved with the device as characterised in claim 1. As a result the controlled disturbance of the magnetic field is substantially independent of the position the elements concerned take up in relation to the magnetic field lines in the magnetic field. The entire device is consequently, as is the object, properly and uniformly visible. As the separate element is, independent of position, visible in the proper manner, the device in its entirety, or at least the section which comprises the element, is clearly visible. It makes no difference if the medical device, when it has an oblong shape, extends in the direction of the magnetic field lines or is at right angles to them. With a uniform distribution of the ferro or paramagnetic material, such an elongated device would be badly visible when it extends in the direction of the magnetic field lines, whereas the disturbance of the field could be too strong when it extends at right angles to the magnetic field lines as a result of which the device could 'outshine' the adjoining areas.

The measure as set out in claim 2 is preferably employed. As a result it is prevented that elements positioned in proximity to one another mutually reinforce the affect on the magnetic field, so that also a resulting directional dependency is avoided.

Additionally, an advantageous embodiment is characterised in claim 3. Preferably the measure as set out in claim 4 is however employed.

A suitable embodiment of the device according to the invention is characterised in claim 5. The body sections can for instance be determined during the extrusion of the basic body by means of the controlled supply of plastic material comprising para and/or ferromagnetic material, alternated with plastic material free of those materials. Another suitable embodiment is characterised in claim 6. Thus it can be ensured in a relatively simple manner that the elements are received in the basic body evenly distributed and at the required distance from one another.

Yet another favourable embodiment is characterised in claim 7. When injection moulding the body, a processing temperature is chosen which is higher than the melting temperature of the basic plastic material of which the body has been made but lower than the higher melting temperature of the plastic material comprising the para and/or ferromagnetic material. Consequently these granules remain received in the matrix of the basic plastic material as separate elements.

Another suitable embodiment is characterised in claim 8. The bore holes can for instance be arranged in required positions by means of laser.

Paramagnetic materials which can be employed in a suitable manner are characterised in claim 9.

Suitable ferromagnetic materials have been characterised in claim 10.

The invention will be explained in greater detail in the following description with reference to the attached drawings, in which a number of examples of embodiments of the device according to the invention have been illustrated.
- Figure 1: shows a partly cut away end of a tube-like device according to the invention.
- Figure 2: shows the cross-section along the line TI-II of figure 1.
- Figure 3: shows a view of another embodiment corresponding to figure 1.
- Figure 4: shows the cross-sectional view along the line IV-IV of figure 3.
- Figure 5: shows yet another embodiment of a tube-like device according to the invention.
- Figure 6: shows a cross-section along the line VI-VI of figure 5.
- Figure 7: illustrates schematically an important aspect of the invention.
- Figure 8: shows a wire provided with elements.
- Figure 9: shows a partly broken away view of an end of a tube-like device according to the invention, wherein the wire of figure 8 has been employed in a reinforcing layer.
- Figure 10: shows a cross-section along the line X-X of figure 9.
- Figure 11: shows a view of an alternative version of an embodiment corresponding to figure 9.
- Figure 12: shows a cross-sectional view along the line XII-XII of figure 11.
- Figure 13: shows a partly cut away view of an end of yet another embodiment.
- Figure 14: shows a cross-section along the line XIV-XIV of figure 13.
- Figure 15: shows a partly broken away view of a device according to the invention in the form of a balloon.

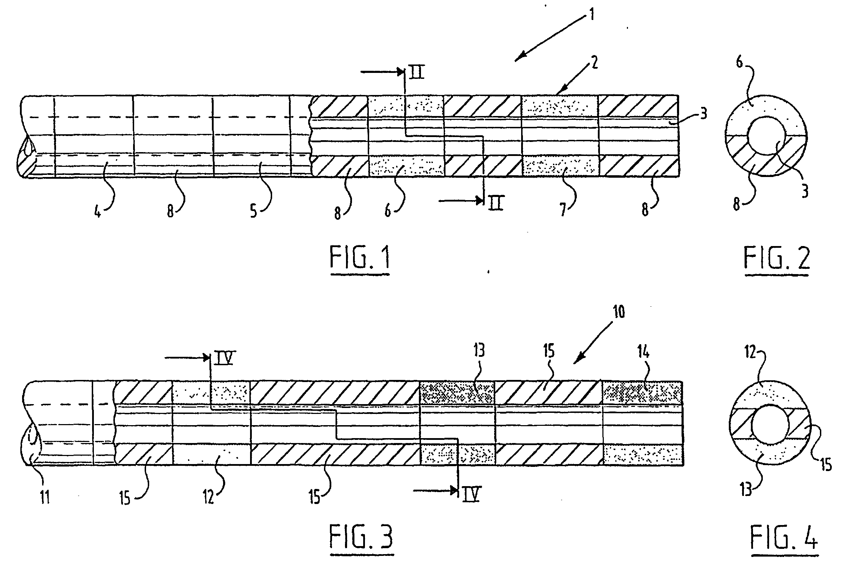

The device 1 as shown in figure 1 is for instance an end-section of the tube-like basic body 2 of a catheter. This basic body 2 comprises a lumen 3 extending through it in a longitudinal direction.

As can be seen in figures 1 and 2, the basic body 2 is made up of a number of body sections 4, 5, 6, 7 comprising para and/or ferromagnetic material alternated with sections 8 which are made of basic material. The sections 4, 7 may for instance have been received in the basic material 8 by means of an injection moulding process. During injection moulding, the supply of basic material 8 and the supply of the material of which the sections 4-7 are made, is in that case switched on and off alternately.

The sections 4-7 provided with para and/or ferromagnetic material have a dimension in the longitudinal direction of the basic body 2, which is substantially equal to the diameter thereof. Thus these sections form elements which have substantially equal dimensions in three directions at right angles to each other.

As a result the disturbance of a magnetic field such as the one used with magnetic resonance imaging (MRI), caused by each of the separate elements, is at least virtually independent of the position these elements take up in this magnetic field. In other words, whether the centre lines of the elements 4-7 run parallel to the magnetic field lines or are at right-angles to them, the disturbance of the magnetic field and consequently the visibility on the screen of the resultant artefact remains more or less the same.

The same is true for the device 10 of figure 3. Also in this case the elements 11, 12, 13, 14 comprising the para and/or ferromagnetic material are alternated with sections 15 made of basic material. The elements 11-14 have also in this case a length which is substantially equal to the diameter.

Additionally the elements 13, 14 which are 5 positioned more towards the end of the device 10, have a greater density of para and/or ferromagnetic material, so that they are more clearly visible on the screen of the MRI-device.

The relative distance between the elements 4-7 of the device 1 and the elements 11-14 of the device 10 is substantially such, that there is at least practically no magnetic interaction between the adjoining elements. With the embodiment of figure 1 the relative distance between the elements is at least equal to the length and the diameter of each of the elements 4-7, which is to say at least equal to the substantially equal dimensions of these elements in three directions at right angles to each other. With a suitable concentration of the para and/or ferromagnetic material in the elements known as such, the relative affect on each other is very small, so that the elements function as separate elements also where magnetic conditions are concerned.

With a stronger magnetic action of the elements, for instance as the result of a certain choice of material or because of a higher concentration of the para and/or ferromagnetic material, a greater distance between the separate elements will be necessary in order to limit the magnetic interaction.

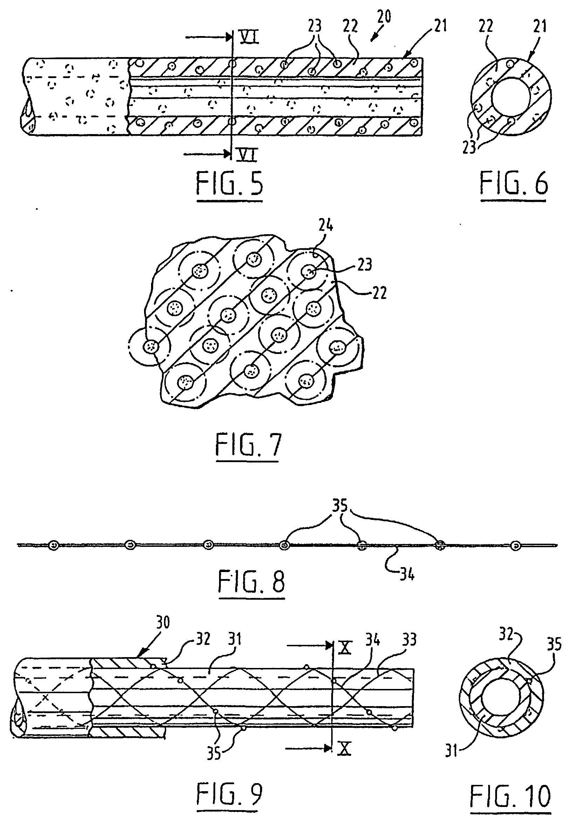

With the device 20 shown in figure 5, the basic material 22 of the basic body 21 is provided with a great number of spherical elements 23 comprising para and/or ferromagnetic material. Because of the spherical shape, the disturbance of the magnetic field is also with these elements independent of the position they take up in the field.

The elements 23 may have been received in the basic material 22 when forming the basic body 21 by means of extrusion or injection moulding. In that case the elements are granules of a plastic material comprising the para and/or ferromagnetic material, which has a melting temperature which is higher than the melting temperature of the basic material 22. Thus the elements 23 can be received as substantially solid particles in a flow of liquid basic material 22. Mixing the particles 23 into the basic material 22, in which case the basic material 22 is also in granular form, can already be done in a cold state. By mixing the different granules properly, an even distribution of the elements 23 in the basic material 22 can be achieved. During the extrusion or injection moulding process, a temperature is chosen at which only the granules of basic material 22 will melt. The granules 23 will remain received in the liquid flow of the basic material 22 in the form of more or less solid particles.

Figure 7 shows schematically the importance of a suitable distance between the elements 23. With the dashed and dotted line 24, an area surrounding each element 23 has been indicated, over which the magnetic field in an MRI device may be considered to be disturbed by this element 23. When the elements 23 would be positioned close to each other, so that these areas 24 would overlap to a significant degree, the two elements 23 concerned would, from a magnetic point of view, fuse together as it were so as to form one elongated element, as a result of which the intended effect would be negated. As can be seen in figure 7, the elements 23 should preferably be arranged at such a distance from one another, that there is no magnetic interaction of significance between the adjoining elements 23.

The invention can also be employed in a suitable manner with a device according to the invention in the form of a catheter provided with a braided reinforcing layer. Such a catheter has been illustrated schematically and in a partly broken away view in figure 9.

The catheter 30 is made up of a first tube-like inner layer 31, which has for instance been extruded around a mandrel which has been removed afterwards. Around this inner layer 31 a reinforcing layer of wires 33, 34 has been braided. For the sake of clarity only two wires are shown in figure 4, but in the usual manner there may be a great number of wires. Next, an outer layer 32 may be extruded around this braided reinforcing layer, so that the reinforcing layer is enclosed by the inner layer 31 and the outer layer 32.

As can be seen in greater detail in figure 8, the wire 34 of the catheter 30 is provided, at equal distances, with elements 35 which are substantially spherical and consequently have equal dimensions in three directions at right angles to one another and comprise para and/or ferromagnetic material. The elements 35 may for instance be arranged around the wire 34 by means of an extrusion process. Another possibility is to attach the elements 35 as drops of hardening material comprising the magnetic material to the wire 34. When the material has hardened, the wire 34 can be incorporated in the catheter 30.

Even when, with the embodiment of figure 9, the elements 35 are positioned so close together that there is a magnetic interaction between the adjoining elements, a good directional independency is achieved after all as the elements 35 will be positioned on a helical line. In particular when this helical line has a pitch angle of 45°, sections of this helical line which are arranged on either side opposite each other will always be placed at right angles to one another, so that there always will be sections of the helical line which are positioned at right angles to the magnetic field.

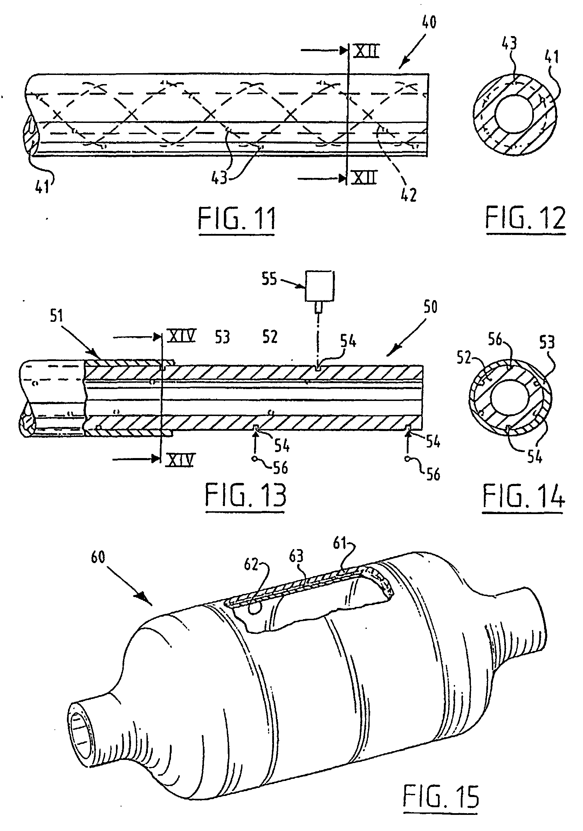

With the device 40 of figures 11 and 12, a braided layer has been co-extruded of which at least one wire 42 carries the elements 43 made of para and/or ferromagnetic material. The device 50 of figure 13 comprises a tube-like basic body 51. This basic body 51 is made up of a tube-like inner body 52, and arranged around it, an outer layer 53. In the tube-like inner layer 52 a number of holes 54 have been formed by means of a laser device. These holes 54 are preferably arranged on a helical line. In the holes 54 formed, elements 56 have been fitted which either comprise or are made of para and/or ferromagnetic material. The elements 56 are temporarily fixed in the holes 54, for instance by means of glueing. Next the inner layer 52, thus provided with elements 56, is passed through an extrusion device and provided with an outer layer 53, which consequently encloses the elements 56 in the holes 54.

The device according to the invention may be any medical device which is received for a shorter or longer period of time inside the body of a patient and of which the position has to be visualized by means of MRI. In addition to tube-like catheters such devices may be stents or grafts.

Another possible embodiment of such a device is a balloon, such as the balloon 60 shown in figure 15. This balloon is arranged on a catheter, and by means of the latter, advanced to the correct position inside the body of the patient.

With the embodiment of figure 15 the balloon 60 comprises an outer layer 61 and an inner layer 62, which layers 61, 62 are connected to each other by means of a layer of glue 63. The layer of glue 63 comprises in this case evenly distributed granular particles of para and/or ferromagnetic material or material comprising such material. By properly mixing the glue and the magnetic material, the elements of magnetic material formed by these granules can be enclosed in between the outer layer 61 and the inner layer 62 of the balloon 60, placed at a suitable distance from one another, so that the balloon 60, independent of the direction of the magnetic field, can be made properly visible when using the MRI device.

Suitable materials of which to make the elements referred to above are for instance paramagnetic materials such as dysprosium and gadolinium and alloys and salts of these materials. Ferromagnetic materials which are preferably used are iron, nickel, cobalt and alloys of these materials. The concentration of the para and/or ferromagnetic material used in the elements may vary from 0.001% in the case of strong ferromagnetic materials to 100% by weight. The concentration of the magnetic material in the elements received in the device can, as described above referring to the figures 3 and 4, vary according to the position of these elements in the device. Thus it is possible to make for instance the distal end-section of a catheter more clearly visible than its more proximally located elements.

## Claims

1. Medical device (1) to be introduced into the body of a patient either temporarily or for a longer period, for use under MRI conditions, comprising a tubular body (2) containing para and/or ferromagnetic material, **characterised in that** the para and/or ferromagnetic material has been received in at least one separate element (6, ..., 63) which has substantially equal dimensions in three directions at right angles to each other.

2. Device as claimed in claim 1, wherein several elements are provided and the relative distance between the elements (6, 7) is substantially such, that there is at least almost no magnetic interaction between adjoining elements.

3. Device as claimed in claim 2, wherein the relative distance is at least equal to the substantially equal dimensions in three directions at right angles to each other.

4. Device as claimed in claim 2, wherein the relative distance is at least equal to twice the substantially equal dimensions in three directions at right angles to each other.

5. Device as claimed in one of the previous claims, wherein the body (2) is tube-like and the elements are body sections (6, 7) of which the length is substantially equal to the diameter.

6. Device as claimed in one of the previous claims, wherein the body comprises a reinforcing layer made of wires (33, 34) and the elements (35) are attached to at least one of the wires (33, 34) at certain distances from each other.

7. Device as claimed in one of the previous claims, wherein the body (21) has been made by extrusion or injection moulding of a plastic material, and the elements (23) incorporated in it are granules of a plastic material comprising the para and/or ferromagnetic material which has a higher melting temperature.

8. Device as claimed in one of the previous claims, wherein the elements (56) have been received in bore holes (54) which have been formed in the wall of the body (51).

9. Device as claimed in one of the previous claims, wherein the paramagnetic material has been chosen from the group comprising dysprosium, gadolinium and alloys and salts of those materials.

10. Device as claimed in one of the previous claims, wherein the ferromagnetic material has been chosen from the group comprising iron, nickel, cobalt and alloys of those materials.

## Patentansprüche

1. Medizinische Vorrichtung (1) zum entweder kurzzeitigen Einführen in den Körper eines Patienten oder über einen längeren Zeitraum zur Verwendung unter MRI-Bedingungen, umfassend einen röhrenförmigen Körper (2), der para- und/oder ferromagnetisches Material beinhaltet, **dadurch gekennzeichnet, daß** das para- und/oder ferromagnetische Material in wenigstens einem separaten Element (6, ..., 63), das im wesentlichen in drei Richtungen unter rechten Winkeln zueinander gleiche Abmessungen hat, aufgenommen ist.

2. Vorrichtung nach Anspruch 1, bei welcher mehrere Elemente vorgesehen sind, und der relative Abstand zwischen den Elementen (6, 7) im wesentlichen derart ist, daß wenigstens nahezu keine magnetische Wechselwirkung zwischen angrenzenden Elementen besteht.

3. Vorrichtung nach Anspruch 2, bei welcher der relative Abstand wenigstens gleich zu den im wesentlichen gleichen Abmessungen in drei Richtungen unter rechten Winkeln zueinander ist.

4. Vorrichtung nach Anspruch 2, bei welcher der relative Abstand wenigstens gleich dem zweifachen der im wesentlichen gleichen Abmessungen in drei Richtungen unter rechten Winkeln zueinander ist.

5. Vorrichtung nach einem der vorstehenden Ansprüche, bei welcher der Körper (2) röhrenförmig ist und die Elemente Körperabschnitte (6, 7) sind, deren Länge im wesentlichen gleich dem Durchmesser ist.

6. Vorrichtung nach einem der vorstehenden Ansprüche, bei welcher der Körper eine Verstärkungsschicht aus Drähten (33, 34) umfaßt, und die Elemente (35) an wenigstens einem der Drähte (33, 34) in gewissen Abständen voneinander angebracht sind.

7. Vorrichtung nach einem der vorstehenden Ansprüche, bei welcher der Körper (21) durch Extrusion oder Spritzguß aus einem Kunststoffmaterial hergestellt ist und die darin beinhalteten Elemente (23) Granulien aus einem Kunststoffmaterial sind, welche das para- und/oder ferromagnetische Material umfassen, das einen höheren Schmelzpunkt hat.

8. Vorrichtung nach einem der vorstehenden Ansprüche, bei welcher die Elemente (56) in Bohrlöchern (54) aufgenommen sind, die in der Wand des Körpers (51) ausgebildet sind.

9. Vorrichtung nach einem der vorstehenden Ansprüche, bei welcher das paramagnetische Material ausgewählt wurde aus der Gruppe umfassend Dysprosium, Gadolinium und Legierungen und Salze dieser Stoffe.

10. Vorrichtung nach einem der vorstehenden Ansprüche, bei welcher das ferromagnetische Material ausgewählt wurde aus der Gruppe umfassend Eisen, Nickel, Kobalt und Legierungen dieser Stoffe.

## Revendications

1. Appareil médical (1) destiné à être introduit dans l'organisme d'un patient, soit temporairement, soit pour une longue durée, afin d'être utilisé en imagerie par résonance magnétique, comprenant un corps tubulaire (2) contenant un matériau paramagnétique et / ou ferromagnétique, **caractérisé en ce que** le matériau paramagnétique et / ou ferromagnétique a été intégré dans au moins un élément séparé (6, ..., 63) qui a des dimensions sensiblement égales dans trois directions à angle droit l'une par rapport à l'autre.

2. Appareil selon la revendication 1, dans lequel plusieurs éléments sont fournis et dans lequel la distance relative entre les éléments (6, 7) est sensiblement telle qu'il n'y a au moins quasiment aucune interaction magnétique entre les éléments attenants.

3. Appareil selon la revendication 2, dans lequel la distance relative est au moins égale aux dimensions sensiblement égales dans trois directions à angle droit l'une par rapport à l'autre.

4. Appareil selon la revendication 2, dans lequel la distance relative est au moins égale au double des dimensions sensiblement égales dans trois directions à angle droit l'une par rapport à l'autre.

5. Appareil selon l'une des revendications précédentes, dans lequel le corps (2) a la forme d'un tube et les éléments sont des sections du corps (6, 7), dont la longueur est sensiblement égale à celle du diamètre.

6. Appareil selon l'une des revendications précédentes, dans lequel le corps comporte une couche de renforcement constituée de fils (33, 34) et dans lequel les éléments (35) sont reliés à au moins un des fils (33, 34) à certaines distances l'un de l'autre.

7. Appareil selon l'une des revendications précédentes, dans lequel le corps (21) a été fabriqué au moyen d'un moulage par extrusion ou par injection d'une matière plastique, et les éléments (23) incorporés dans celui-ci sont des granulés d'une matière plastique comprenant un matériau paramagnétique et / ou ferromagnétique qui a une température de fusion plus élevée.

8. Appareil selon l'une des revendications précédentes, dans lequel les éléments (56) ont été disposés dans des trous tubulaires (54) qui ont été creusés dans la paroi du corps (51).

9. Appareil selon l'une des revendications précédentes, dans lequel le matériau paramagnétique a été choisi à partir du groupe comprenant le dysprosium, le gadolinium et les alliages et sels de ces matériaux.

10. Appareil selon l'une des revendications précédentes, dans lequel le matériau ferromagnétique a été choisi à partir du groupe comprenant le fer, le nickel, le cobalt et les alliages de ces matériaux.
